Europäisches Patentamt
European Patent Office
Office européen des brevets

(19)

(11) Publication number: **0 108 341 A2**

(12) # EUROPEAN PATENT APPLICATION

(21) Application number: 83110706.5

(22) Date of filing: 26.10.83

(51) Int. Cl.³: **A 61 B 5/04**

(30) Priority: 02.11.82 US 438592

(43) Date of publication of application: 16.05.84 Bulletin 84/20

(84) Designated Contracting States: AT BE CH DE FR GB IT LI LU NL SE

(71) Applicant: MYO-TRONICS RESEARCH, INC.
720 Olive Way - Suite 800
Seattle Washington 98101(US)

(72) Inventor: Radke, John Charles
5260 - 16th Avenue, N.E.
Seattle Washington 98105(US)

(72) Inventor: Ryan, Gregory Jerome
12529 - 22nd Avenue, N.E.
Seattle Washington 98125(US)

(74) Representative: Patentanwälte Grünecker, Dr. Kinkeldey, Dr. Stockmair, Dr. Schumann, Jakob, Dr. Bezold, Meister, Hilgers, Dr. Meyer-Plath
Maximilianstrasse 58
D-8000 München 22(DE)

(54) **Mandibular electromyograph.**

(57) A mandibular electromyograph having a plurality of electrodes adapted to generate electromyograph signals indicative of masticatory muscle potentials. The electrodes are selectively connected to a preamplifier by a multiplexer circuit. The preamplifier also generates and applies to an electromyograph preamplifier channel a 60 Hz test signal generated by amplifying the output of an antenna by a 60 Hz selective amplifier. The preamplifier outputs are applied to respective analog processing circuits which provide DC decoupling followed by a 60 Hz notch filter and then a 60 Hz band-pass filter. Analog signals available at various points in the analog circuit are selectively applied to a digital circuit by a second multiplexer. The digital circuit rectifies and then integrates the output of each channel. The integrated output is then converted to a corresponding digital value and applied to a processor. The processor averages several sequential digitized samples and selects them for printing by an internal printer. The digital data is selected for printing in accordance with both the type of test being conducted and the characteristics of the digital data. For example, in a function test, digital data is not printed unless the electromyograph signals vary by a predetermined magnitude within a specific period of time. The processor also applies signals to various indicating lights and it checks the condition of the various switches and controls.

FIG. 1

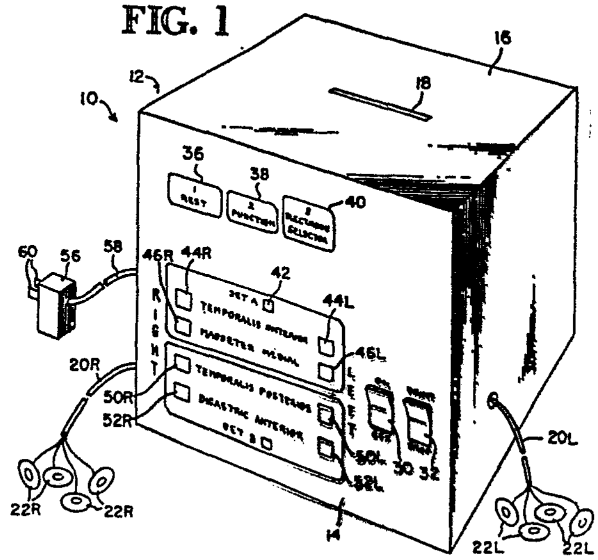


EP 0 108 341 A2

# Description

## MANDIBULAR ELECTROMYOGRAPH

### Technical Field

This invention relates to dental diagnostic instruments, and more particularly, to a mandibular electromyograph for measuring and printing a record of the electrical activity of the masticatory muscles while at rest and while performing specific functions.

### Background Art

Electromyographs utilize conventional electrodes placed on the surface of the skin to pick up electric potentials generated by contraction of the muscles beneath the skin. Electromyographs have long been used in the medical and dental fields for a variety of purposes, including the examination of masticatory muscles for pathological conditions. The masticatory muscles are muscles of the human body which produce mandibular movement associated with chewing.

Although mandibular electromyographs have been previously used, their use is limited by the difficulty of interpreting the information thereby obtained. In the conventional mandibular electromyograph, the electric potentials from the electrodes are amplified, filtered, and then usually applied directly to the X axis of an oscilloscope or a strip chart recorder. The characteristics of a single masticatory muscle are examined by measuring the spacing of various portions of the electrode signal on the oscilloscope screen or strip chart. The accuracy of this technique is inherently limited by the resolution of graticules or scales printed on the strip chart. Furthermore, it requires a great deal of skill and concentration by the practitioner administering the test. The characteristics of masticatory muscle contractions can only be compared to the contractions of other masticatory muscles with a great deal of difficulty. Such comparisons are traditionally made by simultaneously displaying two, four, or more channels, each containing an electrode output, on the oscilloscope or strip chart at the same time. Use of this technique is even more difficult with an oscilloscopes since it is extremely difficult to simultaneously examine multiple traces on the oscilloscope screen. This technique is used with strip chart recorders by manually drawing transverse lines across the strip chart and comparing the recording for each channel with the line. The line drawing and comparing process is, of course, slow and tedious.

The foregoing disadvantages have limited, to some degree, the widespread acceptance of mandibular electromyographs for clinical use. These limitations resulted in the development of an improved electromyograph described in U.S. Patent No. 4,344,441, issued to applicant herein. The '441 patent simplified the understanding of electromyographic data by displaying both a digital value and an analog bar graph of various amplitude and time-related characteristics of an electromyographic waveform. Additionally, the mandibular electromyograph displayed digital indications of the timing relationships between two or more electromyograph signals.

The ability to display various characteristics of electromyographic signals in both analog and digital form greatly facilitated the use of electromyography in dentistry. However, even the device disclosed in the '441 patent is somewhat limited by, for example, the inability to generate a printed record of the electromyographic signals. The difficulty in generating a printed record goes beyond merely sampling the electromyographic signals and printing out their values, for to do so would result in a large number of printed values, thus making interpretation difficult. A mandibular electromyograph capable of printing test values which allow easy interpretation of the test results necessitates discriminating between which samples of electromyograph signals are to be printed. Additionally, the mandibular electromyograph disclosed in the '441 patent could sometimes produce erroneous information without the operator's knowledge of a malfunction if, for example, an electrode is not making proper contact with the patient or a wire to the electrode is open. This disadvantage can be particularly severe, because dental diagnostic instruments are often used by individuals who do not have a great deal of training in the field of electronics. Finally, prior art mandibular electromyograph devices often do not provide circuitry for adequately eliminating noise in the bandwidth of interest, particularly noise introduced by 60 Hz power signals.

Disclosure of the Invention

It is an object of the invention to provide a mandibular electromyograph that periodically samples an electromyograph signal and prints information from only the samples which are most meaningful to a clinical evaluation.

It is another object of the invention to provide a battery-powered mandibular electromyograph that internally generates an AC test signal matched in frequency to that of ambient 60 Hz noise.

It is still another object of the invention to provide an electromyograph that is capable of checking for proper electrode connections and attachment.

It is a further object of the invention to provide a mandibular electromyograph that indicates the mandibular function to be performed and then selects data to be printed depending upon the test performed in order to optimize the usefulness of the printed information.

It is a still further object of the invention to provide an easily understood indication when a person's masticatory muscles are sufficiently at rest for a functional test to begin.

These and other objects of the invention are provided by a mandibular electromyograph in which electromyograph signals indicative of masticatory muscle potentials are generated by a plurality of electrodes. The electromyograph signals are amplified and then applied to a digital-to-analog converter which periodically samples signals derived from the electromyograph signals and generates digital data indicative of the amplitude of the samples. At least a portion of the digital data is then selected for printing by an internal printer. The digital data may be selected as a function of a characteristic of the samples in order to reduce the volume of data printed. This selection may be carried out by comparing sequentially generated digital data and then printing the digital data only when it differs by a predetermined magnitude from digital data derived from an earlier occurring sample. The digital data may be derived from a number of samples, such as by calculating an average of a plurality of sequentially occurring samples. In the event that the samples do not change significantly over a relatively long period, digital data derived from the samples may nevertheless be selected for printing. The digital data may also be selected for printing as a function of the characteristic of the test being performed with the electromyograph. Thus, in performing a resting test, the digital data may be the average amplitude of a plurality of sequential samples. In a functional test, the digital data may be selected for printing only when it differs by a predetermined magnitude from digital data derived from at least one earlier occurring sample. The electromyograph signals may be rectified and then integrated before being applied to the digital-to-analog converter. In such case, the rectifier may be calibrated by applying a DC reference voltage to a circuit upstream from the integrating means and the output of the integrating means can be measured after a predetermined integrating time. The electromyograph may also include a circuit for testing the quality of attachment of the electrodes to a patient and the condition of the electrical leads connecting the electrodes to the amplifier. Basically, the testing means detects for the presence of significant 60 Hz noise on the electromyograph signal which would occur in either of the above-mentioned abnormal conditions. The 60 Hz noise may be detected by applying the electromyograph signal to a 60 Hz filter circuit and then comparing the amplitudes at the input and output of the filter circuit. The electromyograph may also include a test circuit for verifying the correct operation of the 60 Hz filter. Basically, this is accomplished by applying a 60 Hz reference signal to the electromyograph channel and checking for predetermined amplitudes at the input and output of the 60 Hz filter. Insofar as the electromyograph may be battery powered, a 60 Hz signal may not be readily available. Under these circumstances, the 60 Hz signal may be generated by utilizing an antenna adapted to pick up 60 Hz ambient electromagnetic radiation and then amplifying the output of the antenna by a frequency discriminating amplifier centered at 60 Hz.

## Brief Description of the Drawings

Fig. 1 is an isometric view of the mandibular electromyograph.

Fig. 2 is a copy of a printed record of the initial power-up printout and the printout resulting from an at-rest test and a function test.

Fig. 3 is a schematic of the preamplifier portion of the mandibular electromyograph.

Fig. 4 is a schematic of the analog portion of the electromyograph receiving the output of the preamplifier of Fig. 3.

Fig. 5 is a schematic of the digital portion of the mandibular electromyograph receiving the output of the analog portion of Fig. 4.

Fig. 6 is a flow chart of the software for controlling the operation of the mandibular electromyograph.

## Best Mode for Carrying Out the Invention

The mandibular electromyograph 10, as illustrated in Fig. 1, is enclosed in a housing 12 having a front panel 14 and top 16 containing a slot 18 through which paper containing data printout exits. Respective multiconductor cables 20 extend from opposite sides of the housing 12 and terminate in four electrodes 22L,R, respectively, of conventional design. Each of the electrodes 22 contain a marking identifying the location on the patient to which it is attached. The electrodes are attached to the temporalis anterior (Ta), the masseter middle (Mm), the temporalis posterior (Tp), and digastric anterior (Da) positions on the right and left sides of the head of the patient.

The front panel 14 contains a number of switches for controlling the operation of the electromyograph 10. These switches include an ON-OFF switch 30, a STOP/PRINT switch 32 for controlling the operation of the internal printer, a "Test 1" switch 36 for selecting the "At-Rest" test, a "Test 2" switch 38 for selecting a "Function Test" and an "Electrode Selector" switch 40 for selecting as "Set A" the temporalis anterior and masseter middle electrodes, or, as "Set B" the temporalis posterior and digastric anterior electrodes. The switches 36 and 38 are mechanically interlocked so that pressing one all of the way in releases the other switch.

When power is initially applied to the electromyograph 10, the electrodes 22 in Set A are selected by default. Under these circumstances, a light-emitting diode 42 on the panel 14 is illuminated and light-emitting diodes 44, 46 are illuminated in proportion to the amplitude of the signals from the temporalis anterior and masseter middle electrodes, respectively. However, as explained in greater detail hereinafter, the light-emitting diodes have a dead band so that they are not illuminated at all for relatively low signals. Thus, when the patient is sufficiently at rest to begin a functional test, the light-emitting diodes 44, 46 are illuminated only sporadically, if at all. When Set A has been selected either by default or activating the selector switch 40, and the selector switch 40 is once again actuated, a light-emitting diode 48 is illuminated and light-emitting diodes 50, 52 are illuminated in proportion to the signals from the temporalis posterior and the digastric anterior muscles, respectively. Once again, as with the light-emitting diodes 44,46, the light-emitting diodes 50,52 have a dead band so that when the temporalis posterior and digastric anterior muscles are at rest, the light-emitting diodes 50, 52 are illuminated only sporadically, if at all. Actuating the switch 40 thus alternately selects between the Set A electrodes and the Set B electrodes.

The mandibular electromyograph 10 is powered by a conventional DC power supply 56 through a two-wire cable 58. The power supply 56 includes a pair of conventional prongs 60 which plug into a 50-60 Hz, 110-220 volt power outlet. By utilizing an external power supply 56, the same mandibular electromyograph 10 can be used throughout the world in regions having AC power available with varying characteristics, since only the power supply 56 must be specially adapted to such characteristics.

A printout generated by an internal printer and received through slot 18 (Fig. 1) is illustrated in Fig. 2. When power is initially applied to the system by actuating the ON/OFF switch 30, a self-test routine is initiated and, if all circuitry is functioning properly, an "All Systems OK" indication is printed at 70. A noise test is then conducted on each of the four electrodes selected by electrode selector switch 40 and a number corresponding to the 60 Hz noise level on each electrode is printed at 72. A line for the name of the patient is then printed at 74 and a place for the date is printed at 76. The electromyograph then begins performing either the at-rest test or a function test, depending upon which switch 36 or 38 is closed.

Assuming that the function test has been selected, the internal printer prints the test selected at 78, followed by the function to be performed, in this case "RELAX -- CLENCH -- RELAX" at 80. Data indicative of the amplitude of the electromyographic signals from the selected electrodes is then printed from 82 through 84, followed by an average of the data for each electrode printed at 86.

A particularly advantageous feature of the inventive electromyograph is the ability to select specific samples of the electromyograph signals for printing depending upon the nature of the electromyographic signals. Thus, the dental practitioner need not tell the patient to "relax, clench, and then relax" at any specific rate of speed. Regardless of the speed at which the patient performs this function, the electromyograph will print data that is most meaningful to the dental practitioner. Accordingly, the first values are the electromyographic signals from the electrodes while the patient is at rest. Although the electromyographic signals are continuously sampled, no further samples are printed until the patient begins to clench his teeth, thereby causing the electromyographic signals to increase in amplitude. A threshold is automatically established on the basis of the resting values and, when this threshold is exceeded, the average of two sequential samples that are above this threshold is printed as the second data entry. Subsequent samples are averaged in groups of four and compared with subsequent four-sample averages. In the event that any subsequent four-sample average differs by a predetermined amount from the previously printed four-sample average, then that four-sample average is also printed. A typical electromyographic wave form resulting from a "RELAX -- CLENCH -- RELAX" test shows a relatively level relaxed state, a fairly steep rise to a clenched state, a fairly constant level during the clenched state, followed by a fairly steep decline back to the resting level. By printing only four-sample averages that are significantly different from a previously printed four-sample average, the rising and leading edges of the waveform as well as the plateau can be reconstructed. In the event that the patient clenched and then relaxed before ten values of data were printed, the test might never be completed because the electromyograph signals would not change after the muscles return to the resting state. Therefore, if 16 samples are taken and none of the four-sample averages exceeds the previously printed data point by the predetermined value, then the average of such 16 samples is printed.

Although a test for the "RELAX -- CLENCH -- RELAX" function is the only function test illustrated herein, other function tests, such as "swallow" or "chew," could also be implemented, as is well understood by one skilled in the art.

In the "AT-REST" test, selected by actuating the "AT-REST" test switch 36 (Fig. 1), the electromyograph printer first prints the test heading "EMG Test 1" at 90, followed by the designation "MANDIBLE AT REST" at 92. The electromyograph signals for each electrode are then periodically sampled and the averages of 256 samples are then printed for as long as the test continues. The data for these 256 sample groups is printed from 94-96. In the example illustrated in Fig. 2, the patient is initially sufficiently relaxed but then becomes tense for several 256-sample averages. Finally, the patient has relaxed for three 256-sample averages and is thus in a condition for a functional test to begin.

The electromyographic signals from the electrodes 22 (Fig. 1) are received by a preamplifier illustrated in Fig. 3. The two leads from the temporalis anterior electrode and the two leads from the temporalis posterior electrode for each side are applied to a conventional multiplexer 100. Similarly, the two leads from the masseter medial and digastric anterior electrodes for each side are applied to a conventional multiplexer 102. The multiplexers 100,102 also receive a 60 Hz test signal and signal ground. One of the four sets of two inputs of each multiplexer 100, 102 is applied to a respective differential amplifier 104, 106, depending upon the two-bit word applied to the A and B inputs of the multiplexers 100,102. As explained below, in the normal operating mode the B input is high or logic "1". Under these circumstances, a logic "0" A input causes the multiplexer 100 to connect the temporalis posterior electrode to the amplifier 104 while the multiplexer 102 connects the digastric anterior electrode to the amplifier 106. When the A input is low or logic "0", the multiplexer 100 applies the temporalis anterior electrode to the amplier 104 while the multiplexer 102 connects the masseter medial electrode to the amplifier 106.

As explained in greater detail below, during a test mode, the B inputs to the multiplexers 100,102 are low or logic "0". Under these circumstances, an A input of logic "0" causes the multiplexers 100,102 to connect the 60 Hz reference signal to the amplifiers 104,106, respectively. An A input of logic "1" cause the multiplexers 100, 102 to connect their ground inputs to the inputs of the amplifiers 104,106, respectively.

The B control inputs to the multiplexers 100,102 are controlled by a flip-flop 110 of the set-reset variety. The flip-flop reset input is normally held low through resistor 112 but, upon power-up, a low-to-high transition generated by the positive voltage being applied through capacitor 114 resets the flip-flop 110, thereby making the Q output -- also the B input to the multiplexers -- low. At power-on, the negative supply voltage applied through capacitor 116 pulls the set input to flip-flop 110 low. However, capacitor 116 then begins to charge through resistor 118 toward a voltage determined by the resistor divider ratio of resistors 118 and 120. After a predetermined period, the set input to flip-flop 110 rises to the trigger level, thereby setting flip-flop 110 and causing the Q output or B input to the multiplexers 100,102 to go high. Thus, for a relatively short test period during power-up, the Q output causes the B inputs of the multiplexers 100,102 to go low. During the test period, the $\overline{Q}$ output of the flip-flop 110 goes high to produce a 60 Hz reference signal, as descsribed in greater detail below.

The gain of the amplifiers 104,106 is about 500. In order to boost the total gain of the preamplifier to about 13,000, the outputs of the amplifiers 104,106 are applied to respective operational amplifiers 140 having a gain set by respective feedback resistors 142 and some injunction resistors 144 of about 30. The outputs of the amplifiers 104,106 are applied to the amplifiers 104 through an RC network composed of capacitor 146, fixed resistor 148 and potentiometer 150. The potentiometers 150 are adjusted so that the gains of the preamplifier channels are equal to each other. The 500 gain of amplifier 104,106 and the gain of about 30 of the amplifiers 40 results in a potential gain of 15,000. The potentiometers 150 are adjusted so that each of the channels has an overall gain of about 13,000. The capacitor 146, in combination with resistors 148,150, results in a low-frequency breakpoint of about 10 Hz, thereby preventing DC offsets generated by the electrodes from being applied to the amplifiers 140. Capacitors 152, connected in the feedback group of respective amplifiers 140, in combination with respective resistors 142, set the high-frequency breakpoint of amplifiers 140 to about 1,000 Hz. Thus, the preamplifier amplifies the electromyographic signals from the electrodes by about 13,000 within a frequency range of from about 10 Hz to about 1 kHz.

In the normal operating load, the signal at the channel 1 output is either the right temporalis anterior electrode or the right temporalis posterior electrode. The signal at the channel 4 output is either the signal from the left temporalis anterior electrode or the signal from the left temporalis posterior electrode. The signal at the channel 2 output is either the signal from the right masseter medial electrode or the right digastric anterior electrode. The signal at the channel 3 output is from either the right masseter medial electrode or the left digastric anterior electrode. The particular one of two possible electrodes generating the output signals for each channel is selected by the ELECT SEL input (A = either logic "1" or "0") from the digital board described hereinafter.

In the test mode (B = logic "0") occurring upon power-up, the signal applied to all four output channels is either ground or the 60 Hz test signal, depending upon the condition of the ELECT SEL input.

It will be remembered that the mandibular electromyograph 10 is powered by an external DC power supply 56 (Fig. 1), thus making an internal AC signal unavailable in the absence of a dedicated 60 Hz signal generator. In order to generate a 60 Hz test signal having a frequency exactly matched to the frequency of ambient 60 Hz noise, the ambient 60 Hz noise is picked up by an internal antenna 200. The output of the antenna is amplified by a high gain operational amplifier 202 having resistor 204 connected in its feedback loop and its noninverting input connected to ground through resistor 206. The output of the amplifier 202 is applied through resistor 208 to a high gain amplifier 210 having positive feedback provided by resistors 212,214. The high gain of the amplifier 210, coupled with the positive feedback, causes the output of the amplifier 210 to be a 60 Hz square wave exactly matched to the frequency of the ambient 60 Hz noise. This 60 Hz square wave is applied through resistor 216 to a pair of back-to-back diodes 218 which clip the square wave at approximately $\pm$ 0.7 volt.

The clipped 60 Hz square wave across diodes 218 is applied to a 60 Hz band-pass filter 220 of conventional design. The band-pass filter 220 is formed by a high gain operational amplifier 222 having a variable gain set by fixed resistor 224 and potentiometer 226 and frequency characteristics determined by capacitors 228,230 in combination with the resistors 224,226. Feedback is also provided through resistor 234, and the signal is applied to the band pass amplifier through a resistor 236. Resistor 238 is provided to reference the input and feedback to ground. The output of the amplifier 222 is inverted by a unity gain inverting amplifier formed by operational amplifier 240 having a feedback resistor 242 equal to that of a summing junction resistor 244. The noninverting input to the operational amplifier is connected to ground through resistor 246.

Because of the narrow band-pass operation of the band-pass amplifier 220, the amplifier 222 outputs a relatively pure 60 Hz sine wave. This sine wave is inverted by amplifier 240 and applied to the multiplexers 100,102 through a potentiometer 204 which controls the level of the 60 Hz test signal applied to the amplifiers 104,106. The amplifiers 210,222,240 are all contained in a single integral circuit package. Thus, all of the amplifiers 210,222, 240 receive power only when the Q output of flip-flop 110 is high. The $\overline{Q}$ output of flip-flop 110 is high only during the test period of the electromyograph occurring upon power-up. As a result, the 60 Hz test signal is not produced during normal operation of the electromyograph, thus preventing the 60 Hz test signal from possibly interfering with the operation of the electromyograph.

The relatively low level of the signals applied to the preamplifier and the high gain of the amplifier make it important to isolate the preamplifier from the remaining circuitry of the electromyograph. For this reason, the preamplifier is powered by two internal 9 volt batteries 260, 262, which generate + and - 9 volts, respectively. The batteries are applied to respective contacts of a relay 264 which is controlled by an external RELAY input. During operation of the electromyograph, the relay 264 is energized in order to apply $\pm$ 9 volt power to the circuitry of the preamplifier. However, when a test is not being conducted, the RELAY input goes low, as explained below, thereby conserving the power of the batteries 260,262 by disconnecting them from the preamplifier circuitry.

The four-channel outputs of the preamplifier are applied to analog circuitry illustrated in Fig. 4. The circuitry includes four identical analog processing circuits 300,302,304,306, only one of which (300) is described in detail herein. The inputs are applied to the noninverting input of an operational amplifier 308 through an RC circuit consisting of capacitor 310, fixed resistor 312, potentiometer 314, and input resistor 316. Capacitor 310, in combination with resistor 312 and potentiometer 314, results in a low-frequency rolloff of about 10 Hz. The potentiometer 314 is adjusted to adjust the overall gain of the analog processing circuit 300. The operational amplifier 308 has a light-emitting diode 318 in its feedback loop that forms part of an opto-isolator, also consisting of phototransistor 320. Light-emitting diode 318 is biased to its linear region by a current flowing through resistor 322 and potentiometer 324. Thus, the collector current of the transistor 320 is proportional to the amplitude of the signal applied to the channel input. A corresponding voltage is thus developed across load resistor 326 and AC-coupled to resistor 328 through capacitor 330. Capacitor 330 and principally resistor 328 form a high-pass circuit having a rolloff at about 10 Hz. The signal across resistor 328 is applied to a dual "one-of-four" multiplexer 332 and to a 60 Hz notch filter 334 of conventional design. The notch filter includes an operational amplifier 336 operating as a voltage follower and a resistor/capacitor network including variable capacitor 338 and potentiometer 340 which are adjusted to adjust the center frequency of the notch filter to 60 Hz. The values of the resistor/capacitor network are calculated in accordance with known principles to one skilled in the art to result in a high Q notch filter which, when capacitor 338 and resistor 340 are appropriately adjusted, is centered at 60 Hz.

The output of the amplifier 336 is, once again, applied to another input of the multiplexer 332 and to a 60 Hz band-pass filter 350, also of conventional design. The band-pass filter 350 includes an operational amplifier 352 and a resistor/capacitor network including a potentiometer 354 that is adjusted to control the center frequency of the band-pass filter 350. The output of the amplifier 352 is applied to the summing junction of another operational amplifier 356 through resistor 358. The noninverting input is grounded through resistor 360, and a feedback network consisting of capacitor 362, resistor 364 and potentiometer 368 is connected in the feedback loop. The capacitor 362, in combination with the resistor 364 and potentiometer 368, results in a high-pass filter having a breakpoint of about 1.6 kHz. The gain of the amplifier 356 is adjusted by adjusting potentiometer 368. The output of the amplifier 356 is also applied to another input of multiplexer 332.

In a similar manner, the corresponding three outputs from the channel 2 analog processing circuit 302 are applied to the multiplexer 332 and the corresponding three outputs from the channel 3 and channel 4 analog processing circuits 304,306, respectively, are applied to another "one-of-four" multiplexer 370. One input on each section of the multiplexers 332,370 also receives a DC voltage reference, the DC voltage supplying power to the circuitry. The particular one of the four inputs connected to the CH1-CH4 outputs is determined by the X,Y control inputs applied to the A and B inputs of the multiplexers 332,370. The outputs of multiplexers 332,370 are normally floating except when their $\overline{\text{ENABLE}}$ input goes low, at which time the selected input for the respective multiplexers 332,370 are applied to their outputs.

The multiplexers 332,370 allow any one of the three outputs of the analog processing circuits 300-306 to be applied to the corresponding channel outputs. The first output, appearing across resistor 328, is the electromyographic signal from the selected electrode amplified and very broadly filtered so that the components of the electromyographic signal from about 10 Hz to about 1 kHz are present. The second input, at the output of the amplifier 336, is the electromyographic signal with 60 Hz noise components substantially attenuated. The output of the amplifier 356 then attenuates all components except for the 60 Hz components. By comparing the amplitude of the input to the band-pass filter 350 (the second output of the analog processing circuit 300) to the output of the band-pass filter 350 (the third output of the analog processing circuit 300), the 60 Hz noise component of the electromyographic signal can be determined. If the electromyograph signal contains relatively little 60 Hz noise, the second output of the analog processing circuits 300-306 will be substantially greater than the third output. However, if the electromyograph signal contains a substantial 60 Hz noise component, the second and third outputs of the analog processing circuits 300-306 will be substantially equal to each other. This comparison is made during the self-test check during power-up, as explained above. The electrode attachment and wire are checked during the noise test subsequent to the self-test mode. If an electrode is not properly attached to the patient or if a wire from an electrode is open, a substantial 60 Hz noise component will be in the electromyograph signal received from the electrode. The operation of the notch filter 334 and band-pass filter 350 is checked in the self-test mode upon power-up by applying the 60 Hz test signal (described above) to each of the analog processing circuits 300-306.

The multiplexers 332,370 also allow a DC reference voltage to be connected to the CH1-CH4 outputs to verify operation of a subsequently described integrator during the self-test procedure. The DC reference voltage is applied to the integrator, and the output of the integrator is checked after a predetermined period to verify that the integrated voltage is at a predetermined value at that time.

The CH1-CH4 outputs from the analog circuitry illustrated in Fig. 4 are applied to a digital circuit illustrated in Fig. 5. The analog signals are applied to respective rectifying circuits 400-406, only one of which (400) is explained in detail. Basically, the analog signals are applied to one input of a "one-of-two" multiplexer 408 across resistor 410 and to an inverting operational amplifier 412 having unity gain as determined by feedback resistor 414 and summing resistor 416. The output of the amplifier 410 is applied to the other data input of the multiplexer 408 so that the multiplexer 408 receives the analog signal at one input and the inverse of the analog signal at the other. The inverted analog signal is also applied to the negative input of a comparator 418 through resistor 420. The inverted analog input is compared to the grounded positive input of comparator 418 so that the comparator 418 generates a positive signal when the analog input is positive and a negative signal when the analog input is negative. The output of the comparator 418 is inverted by inverter 422 so that the output of inverter 422 is positive when the analog input is negative. The output of the comparator 418, when positive, connects the output of amplifier 412 to the output of the multiplexer 408. Similarly, when the output of inverter 422 is positive, the analog input is connected to the output of the multiplexer 408. The output of the multiplexer 408 is thus the negative, full-wave rectification of the analog input.

The rectifier 400 outputs to an integrator 430 formed by an operational amplifier 432 having a capacitor 434 connected in its feedback loop and the series combination of resistor 436 and potentiometer 438 as a summing resistor. The potentiometer 438 is adjusted to control the integration rate of the integrator 430. When a negative signal is applied to the integrator 430 by the rectifier 400, capacitor 434 begins charging so that the voltage across the capacitor 434 is the integral with respect to time of the inverted voltage at the output of the rectifier 400. The integrator 430 is reset to zero by an electronic switch 440 connected across the capacitor 434 and controlled by the eleventh output port of a microprocessor (described below). The integrated output of the processing circuit 400, as well as the outputs of the processing circuits 402-406 for the other channels, are applied to a "one-of-eight" multiplexer 480.

The multiplexer 480 also receives inputs indicative of the voltage from the + and - 9 volt batteries powering the preamplifier. The + and - 9 volt batteries are connected through respective switches 490,422 to each other through a series combination of potentiometer 494, resistor 496, light-emitting diode 498, light-emitting diode 500, resistor 502, and potentiometer 504. The light-emitting diodes 498,500 form part of respective opto-isolators including respective phototransistors 506,508. The phototransistors 506,508 are connected as emitter followers across respective emitter-follower resistors 510,512. The voltage on the emitter of transistors 506,508 is thus proportional to the current flowing through the light-emitting diodes 498,500, which is principally determined by the resistance of resistor 496, potentiometer 494, resistor 502, and potentiometer 504. The potentiometers 494,504 are adjusted to bias the phototransistors 506,508 so that they output a predetermined voltage when the batteries are at their proper voltage. The switches 490,492 are closed by the TEST signal from the $\overline{Q}$ flip-flop 110 (Fig. 3) during the self-test mode upon power-up. Thus, when power is initially applied to the electromyograph, voltages indicative of the voltages of the + and - 9 volt batteries 260,262 (Fig. 3) are applied to the multiplexer 480.

One of the analog voltages applied to the inputs of the multiplexer 480 is applied to the input of a conventional analog-to-digital (A/D) convertor 520 which generates an eight-bit word indicative of the amplitude of the analog signal. The digital word is applied to a conventional microprocessor 530 having an eight-bit data bus D0-D7, a read output $\overline{R_D}$, a data ready input $T_0$, a write output $\overline{WR}$, a timing input $T_1$, a PROG output, and 27 output ports P1-P27. Additional output ports P40-P43, P50-P53, P60-P63, and P70-P73 are generated by a conventional expansion port circuit 534 from the P20-P23 ports and the PROG output of the microprocessor 530. Basically, the PROG outputs a bit to an output port of the expander circuit 534 that is designated by the P20-P23 outputs of the microprocessor.

The analog-to-digital convertor 520 begins operating when the P43 port of the expander circuit 534 outputs a low to the $\overline{WR}$ input of the A/D convertor 520. The amplitude of the signal applied to the A/D convertor 520 is then measured and converted to a corresponding digital value. When the conversion has been completed, the A/D convertor 520 outputs a bit to the $T_0$ input of the microprocessor 530, and the microprocessor 530 then responds by outputing an $\overline{R_D}$ low to read the eight-bit digital word into the microprocessor 530.

The microprocessor 530 also performs a number of other functions. For example, the microprocessor 530 outputs data to a conventional printer 540 inside the housing 12 (Fig. 1) through a conventional output latch 542. The microprocessor 530 first outputs the data on the data bus D0-D7 and then generates a negative-going write pulse $\overline{WR}$ to latch the data on the data bus into the latch 542. When the printer BUSY output goes low to inform the microprocessor 530 that it is ready to accept data, the microprocessor 530 generates a DATA STROBE pulse on its P27 output port to transfer the data from the latch 542 into the printer 540.

The microprocessor 530 also drives the light-emitting diodes 42-52 on the front panel 14 (Fig. 1) through the expander circuit 534. Basically, the light-emitting diodes 42-52 are connected in series from the + 5 volt supply through respective resistors, indicated generally at 550, and respective drivers, indicated generally at 552. The microprocessor 530 periodically energizes either light-emitting diode 42 (designating the Set A electrodes) or light-emitting diode 48 (designating the Set B electrodes). The microprocessor 534 also pulse-width modulates the light-emitting diodes 44-52 in proportion to the intensity of the electromyograph signal received from the corresponding electrodes 22. As mentioned above, however, the microprocessor 530 implements a dead band by not illuminating the light-emitting diodes 44-52 until the intensity of the electromyograph signals exceeds a predetermined threshold.

The relay signal for applying 9 volt power to the preamplifier circuitry (Fig. 3) is also generated by the microprocessor 530. Accordingly, the microprocessor 530 generates the low-level $\overline{\text{RELAY}}$ signal to apply power to the preamplifier by generating a low at its P10 output port. Inverter 560 then saturates transistor 562 through resistor 564, thereby causing current to flow through relay coil 264 (Fig. 3) and close the relay contacts connected to the + and - 9 volt batteries 260,262. A high at the P10 output port of the microprocessor 530 cuts off transistor 562, thereby deenergizing the relay coil 264.

As mentioned earlier, the P11 output port of the microprocessor 530 closes the switches 440 to discharge the integrating capacitors 434 of the integrators 430. The P12 output port of the microprocessor 530 generates the $\overline{\text{ENABLE}}$ signal applied to the multiplexers 332,370 (Fig. 4) for outputing the analog signals from the analog circuit board. The P17 output port of the microprocessor 530 is applied to an opto-isolator through resistor 572. The output of the opto-isolator is connected between - 9 volts and the ELECT SEL input to the multiplexer 100,102 (Fig. 3) that select either the "Set A" or "Set B" electrodes to be output by the preamplifier. The multiplexer 480 is also controlled by the P20-P23 of the microprocessor 530 through the expanded circuit 534. Finally, the P14-P26 ports of the microprocessor 530 receive inputs from the TEST SELECT switches 36,38, the electrode selector switch 40, and the PRINT/STOP switch 32 (Fig. 1). All of the switches are biased high through resistors 580, and the other terminals of the switches are grounded. The PRINT/STOP switch 32 is moved either up or down to ground either the P14 or P15 input port of the microprocessor 530. The electrode selector switch 40 is of the push-push variety, in which the switch changes position to either float or ground the P24 input port each time the switch 40 is actuated. As mentioned earlier, the test selector switches 36,38 are mechanically interlocked so that closing one switch normally opens the other switch so that only one input port P25,P26 is normally grounded at the same time. However, both switches can be closed by pressing both simultaneously and both switches can be opened by pressing one of the switches in slightly and then releasing it. Thus, four possible states of the switches 36,38 are possible so that four different tests can be selected, although only two tests are used in the embodiment disclosed herein. The microprocessor-based digital circuitry thus receives a digital word indicative of various analog signals, outputs data to the printer 540, illuminates the light-emitting diodes 42-48, tests the position of the front panel switches 32-40, and generates a number of control signals used for controlling the operation of the electromyograph 10.

Insofar as the electromyograph 10 is controlled by a microprocessor 530, it operates in accordance with a program of instructions described in the flow chart of Fig. 6. Processing starts at 600 upon power-up and proceeds to 602, where various housekeeping functions are performed, as is well known to one skilled in the art of programming. For example, various buffers are cleared and flags set. At 604, the self-check procedure is initiated in which the following functions are performed:

(1) The ± 9 volt batteries are checked by closing switches 492,490 (Fig. 5) and sequentially applying the voltages at the emitters of transistors 506,508 to the A/D converter and then checking the corresponding digital outputs. If the voltage is below a predetermined value, the instruction "REPLACE BATTERIES" is printed by the printer 540.

(2) The DC reference 332,370 (Fig. 4) is switched to the CH1-CH4 outputs, integrated by the integrators 430 and checked after a predetermined period for the proper voltage level.

(3) The zero volt references are switched to the inputs of the amplifiers 104,106 (Fig. 3) by the multiplexers 100,102 and the digital word from the A/D converter 480 is checked for each channel to verify a low-level condition.

(4) The 60 Hz reference signal is applied to the amplifiers 104,106 (Fig. 3) by the multiplexers 100,102, and the three outputs of each analog processing circuit 300-306 are sequentially switched to the A/D converter 520 (Fig. 5) and checked for the proper levels, thereby verifying the preamplifier gain, notch filter operation, and band-pass filter operation.

(5) A scale factor for each of the four analog channels is calculated on the basis of the previously conducted gain measurements and the scale factor subsequently used to determine the true amplitude of the electromyograph signals.

(6) The microprocessor 530 generates an ELECT SEL signal to cause the multiplexers 100,102 (Fig. 3) to connect the "Set A" electrodes to the amplifiers 104,106.

(7) Assuming that all of the above-described tests are satisfactory, the printer 540 prints "ALL SYSTEMS OK."

(8) After the electrodes have been attached to the patient, the Set A electrodes are connected to the channels CH1-CH4, and two outputs of each analog processing circuit 300-306 are checked to determine the amplitude of the 60 Hz noise on the electromyograph signal. The "Set B" electrodes are then connected in the same manner and the test is repeated. If the 60 Hz noise component is above a predetermined value, the printer 540 prints "POOR ELECTRODE CONNECTION X", where "X" identifies the electrode having the poor connection.

After the self-check test is completed, the program proceeds to 606, where the introduction, including the space for the name of the patient 74 (Fig. 2) and date 76, is printed. The test switches 36,38 are then tested at 608 and, if the switch 36 is found at 610 to have been selected, the program proceeds to 612, where the Test 1 heading is printed as in 90 (Fig. 2). The electromyograph channels for the selected electrodes are then periodically sampled at 614, and the microprocessor then calculates averages of each successive 256-sample averages. The condition of a print flag (reset if printing is to occur, as explained below) is then checked at 615. If the print flag is reset, the average value of these 256 samples for each of the four selected electrodes is then printed at 616 before returning to 608, where the test switches 36, 38 are once again scanned.

If the function test switch 38 is found at 610 to have been selected, the program proceeds via 618 to 620, where the heading for Test 2 is printed as at 78 (Fig. 2). The electromyograph signals for the four selected electrodes are then stored while the patient is at rest at 622 in order to establish a base line and to calculate a threshold. Subsequent samples are then checked at 624 to check for a sample exceeding the threshold. If the program determines at 626 that the threshold is not exceeded, the program repetitively loops through 624 until the threshold is exceeded. When the threshold is finally determined at 626 to be exceeded, the program proceeds to 628, where a four-sample average is calculated and stored and a new threshold is established. Thereafter, the program rechecks at 630 to determine if the update threshold is exceeded by subsequent samples. If the program determines at 632 that the updated threshold has been exceeded, an average of the four subsequent samples is calculated and stored at 634 and the threshold is updated. The program then checks at 636 to determine if the previously stored average is the tenth such average. If not, the program returns to 630, where the samples are once again checked to determine if the updated threshold is exceeded. If, after eight samples, the threshold has not been exceeded, a default is detected at 632 and the average of the previous eight points is calculated and stored at 634 in the same manner as if the threshold had been exceeded. In this manner, averages of four samples exceeding a threshold or eight samples in a default condition are stored until ten such averages have been stored and detected at 636. Thereafter, the condition of the print flag is checked at 637. If the print flag is in a reset condition, the printer 540 prints the ten samples for each channel at 638, calculates the average of the ten points for each channel, and then prints these averages at 640. The program then returns to the main loop via 642, where the test switches 36, 38 are once again scanned at 608.

The microprocessor 530 (Fig. 5) contains an internal interrupt subroutine which interrupts the microprocessor 530 every 20 milliseconds. Thus, every 20 milliseconds, an interrupt subroutine is entered at 650. In order to keep track of the elapsed time of the test, timer variables are updated every 20 milliseconds at 652 and the light-emitting diodes 42-48 and 44-46 or 50-52 are illuminated at 654. It will be remembered that the light-emitting diodes 44-52 are pulse-width modulated. The microprocessor 530, in illuminating the light-emitting diodes 44-52 at 654, thus controls the length of time that the light-emitting diodes 44-52 are illuminated depending upon the amplitude of the corresponding electromyograph signal. The program then checks the condition of the PRINT/STOP switch at 656. If the switch 32 has been actuated downwardly to indicate a stop, the print flag is set at 657 and the program returns through 658 to the main program. If the PRINT/STOP switch 32 has not actuated downwardly, the program checks at 660 to determine if the switch 32 is actuated upwardly. If so, the print flag is reset at 659 and the program returns to the main program via 658.

Although the flow chart illustrated in Fig. 6 can be implemented with a variety of instructions with relative ease by one skilled in the art of microprocessor programming, a set of object codes for one such program is as follows:

0200000014001A
:10070000E5EEFA0E016EEEE018E0CFE03015734
:10071000FEBFFC14C0EFF514733F101445EF225A45
:10072000E1140122096255EE3FF0C593B8205B04E6
:10073000EF00F0186BFFF7AFEB3FF3D0445420 3F5
:100740002E54012210AA42DA564D83E905850 46FBD
:10075000149E240E2B49E8960140E94B4B40299F785
:1007600001E5E6E9005BE3FA0E93DF0C596E75B49C
:10077000E2E492457A150E9828E8221C5567C9A7F2E
:100780005EE3FF0C525B3755E6E908A804E94565D
:10079000E5A7F2553755FD455708B20237B94A572
:1007A0004B1FF07437E94A5B4A0D55B3E0A53F041
:1007B0000C5E6BD2457EE51E0A018E1A1EFA99459
:1007C0A12553530F76D4AED4232004D303E0E3D8
:1007D049E5B469F6EE1E37C6E637146804DB2368
:0E07E0005EEB04F0A11918EEEB8355
:100800000E75E2A055144EEE04B42EB83DE0089B2C53
:1008100000E00395F75A7FD5EC32EDCCFEE0C5231410
:1008200062552539E0AE370A9E9014D2230014A5
:10083000652C4B4C45E2251405230014582 32AB415
:100840004B40814520424E0E3B14DEB404235FB43D
:1008500042434E3C6534805B1442179659 4217C63B
:100860005CEB55A6B3E537CA0F7F7F7F67044 90FC
:10087000E901540D4BD642E59F7D4EFB415D4201F
:10088098B4B4B4269 57EE9A5E4159 4B4D42696 7647
:100890E9E8E40B247689049FD42FEB3F5F6A4F2
:1008A002DFE4EE2420E06A4F920FB16D0BD0E9DC8
:1008B020FCA8L5F9C549F0A9EF0415F018D400EB
:1008C03DFEA119C5EFEB22F969A214E7B92DF09DC9A
:1008D042DE950895FB3AF5EE045874B92EF0C65D
:1008E0EFF1832037EEF6F5F002203761FEF518DC
:0708F0001EEDE24F6B5B3D4
:10090000B9025 4D2B822940B48E932D808385E428
:1009100043B37414E7D5B940B826B462B49EB465EF
:100920003A077626441C05B874B4A2B87414E7BA9F
:1009300009B426C09B44234D7CC7E3F1CEC3523A5
:100940F86C371797F797F735C5A119BF04B83071
:10095003F0A11918F0A11819EF50F903F7A9C52555
:10096000E92C80FFEA313496D46FD420BA9AD5E980
:1009700040C5284283805F119C5AED457E5769460
:1009800064EA72BA9B4968944B830BB04F150A09C
:1009900018277 0A01819EB8DEA09BB04B909B8309A
:1009A00FB19D438B0C0A01919EEA0B830EB04F8
:1009B00F0EED457EB5023 2DB4C45454232DB4C402
:1009C0004435E404E9D0272C9CE4099801B45FE880
:1009D0014D0F90750E314E9B4095E800B4AFB419
:0509E000FCE8D9A33FC
:100A000000C4C594F20E4524F4E494352052 45D0
:100A10005345415243430202045402D22 2053451E
:100A2000414E2042454F4D45FF2020204140402055
:100A30005E5445440E204F45FF20202055041DB
:100A4000544F454E4E452044E414D4E1C22020FF16
:100A500045414720544555542DFF20204041454E5
:100A60034414C45204154 42052455254FF202022FF
:100A70000E4F425345204E455254 0FF202013
:100A800024547495442022020204C454E5402F7
:100A900020544120222040402202041 4D202054
:100AA0054410FF20120524547495420202 04E
:100AB00202040454E54 0202054502020204441FC
:100AC002020444120202054500FF4540415016
:100AD005E45442054454045 0DFF20404945 2EFF95
:0A0AE0001323343536373839C5
:10040000F5A337C6E37146B1E6439E3204355 4072
:100410004DF54C41544256452043 4C454E4346FFA9
:10042000E524540415320434C454E4246205 245AC
:10043000C415800FF454149 4C5552452023FF433E
:100440042E2045 4C454354 524F444E2023FFF2
:100450000044 9E2047524F554E442045 4C454 270
:10046005E4F4445FF23444154452035F5F2FA4
:10047005F5F2F5F5E0FFE4EE6C14EDDFEC5FE
:10048000A9EDF947A514C0AEE514CEE09A14EFE
:1004900A49A5F114CA15F147140FF1530F0CED07
:1004A00EE14691923C5EF79C4C03EDE319F113EE
:1004B0EF115572320145E22E452E4C0949297
:0E04C000E14C140E945284CE45223C0945EC
:100500000EE2BA42A5B01A409B680E2ED14E2230EB9A
:100510014E9F984551595E42250FE5DFB4347E049
:10052000E1E2315EB4F2EEC8457 1E8E2593 2CA9
:100530012ED49E5216349514AC46FE51EE642FC
:1005400C4413E849E547EE47EB47EB47EEE24E1E6
:1005500E3974FB57EFDEF2572F672F67A119196
:100560015EE5E29E49EE47EE47EE2459755945
:10057074F257C5F057A115181EEE718334EEE
:1005800149CD46FA49EE2A28274EB04F119E4A59
:100590016277B4818EEC52E274E0E27A01EE541
:1005A039E6E33EDEB804F0A119 1EEBAC525232 37F
:1005B0F14BEF344EE92 9BF4827A119EFFB8337
:0B05C0094D04DEE914146EEB0E83CD
:100600012E0937E937F5EE57C4245997 29F72E8
:100610F7EE1837E937C42E27E937EE2259D42 1FF
:1006200012ECBC9729E3E5534B0828D2 33E52EDA
:1006300574B9 0279E2A5C43EB87E04DEE8EB14E5
:1006405E34B45B4D530F257F015205 30F6B476C
:10065005AED457EB47E25FEEB1EE5C8FEF7EE51
:10066FC7C57ACFD7EA0EF5D901 5FC0A918E5924
:100670E2EDFEDB041C57EB2E2C9 7E5272E1E9EE
:1006800E4BF23CA14EEFEE2DD53FBCE9ZEFFF9
:100690249E0F025EC0B2 24EEC57C4C0B0C0EEFD
:1006A0EEDF8C4EC05F0C2E0C90094778E1EEE3
:1006B0E7F9BF7F7F7EEEA2476440A0E0C2 47FF
:0206C000FFFF59
:00000001FF


BAD ORIGINAL

Claims

1. A mandibular electromyograph comprising:

a plurality of electrodes (22) adapted to be attached to a patient at locations which cause said electrodes to generate said electromyograph signals indicative of masticatory muscle potential;

amplifier means (104,106,140,308) for selectively amplifying said electromyograph signals;

digital-to-analog converter means (520) for periodically sampling a signal derived from said electromyograph signal to generate digital data indicative of the amplitude of said samples;

processing means (530,534) for selecting digital data indicative of specific samples of said electromyograph signals; and

printer means (540) for printing digital data selected by said processing means.

2. The electromyograph of claim 1 wherein said processing means (530,534) selects said digital data as a function of a characteristic of said samples, thereby reducing the volume of digital data printed by said printer means.

3. The electromyograph of claim 2 wherein said processing means (530,534) selects for printing digital data derived from one of said samples when said digital data differs by a predetermined magnitude from digital data derived from at least one earlier occurring sample.

4. The electromyograph of claim 3 wherein said processing means (530,534) derives said digital data as an average of the amplitudes of a plurality of sequentially occurring samples.

5. The electromyograph of claim 3 wherein, in the event that digital data derived from at least one of said samples does not differ by said predetermined magnitude from digital data derived from at least one earlier occurring sample after a predetermined number of samples have been taken, said processing means (530,534) selects said digital data for printing regardless of the magnitude of said digital data.

6. The electromyograph of claim 1 wherein said processing means (530,534) selects said digital data as a function of the characteristic of the type of test being performed with said electromyograph.

7. The electromyograph of claim 6 wherein, in a resting test, said processing means (530,534) selects as said digital data the average amplitude of a plurality of sequential samples.

8. The electromyograph of claim 6 wherein, in a functional test, said processing means (530,534) selects for printing digital data derived from at least one of said samples when said digital data differs by a predetermined magnitude from digital data derived from at least one earlier occurring sample.

9. The electromyograph of claim 8 wherein, in the event that digital data derived from at least one of said samples does not differ by said predetermined magnitude from digital data derived from at least one earlier occurring sample after a predetermined number of samples have been taken, said processing means (530,534) selects digital data for printing regardless of the magnitude of said digital data.

10. The electromyograph of claim 6 wherein said processing means (530,534) causes said printer means (540) to print the type of test being performed before processing said samples in accordance with a characteristic of the samples expected in the type of test being performed.

11. The electromyograph of claim 1, further comprising:

rectifying means (400,402,404,406) receiving the output of said amplifier means (104,106,140,308) for generating rectified electromyograph signals; and

integrating means (430,402,404,406) for generating and applying to said digital-to-analog converter means (520) integrals with respect to time of said rectified electromyograph signals.

12. The electromyograph of claim 11, further including a calibration circuit comprising:

DC reference voltage means (260,262) for applying a DC reference voltage to said integrating means (430,402,404,406); and

measurement means (520,534,530) for measuring the magnitude of said integrating means (430,402,404,406) and comparing said output to a predetermined value in order to verify the correct operation of at least a portion of said electromyograph.

13. The electromyograph of claim 1, further including electrode testing means for checking the quality of said electrodes to said patient and the condition of electrical leads connecting said electrodes to said preamplifying means, said electrode testing means comprising:

a filter circuit (334,336) receiving respective signals derived from said electromyograph signals, said filter circuit (334,336) having a transfer function that has a magnitude substantially different at 60 Hz than at adjacent frequencies; and

means for comparing (520,530) the amplitudes of the input and output of said 60 Hz filter circuit (334,336) so that an improper attachment of said electrodes (22) to said patient and an open electrical lead from an electrode results in a relatively high, 60 Hz noise component in said electromyograph signal that is detected as a relatively large difference in the amplitudes of said filter circuit (334,336) input and output.

14. The electromyograph of claim 13, further including circuit test means for verifying the correct operation of said electrode testing means, said circuit test means comprising:

a 60 Hz reference signal generator (200-220) producing a 60 Hz test signal, said test signal being applied to the input of said filter circuit (334,336); and

measurement means (520,530) for measuring the magnitude of the input and output of said filter circuit and comparing said input and output to respective predetermined values in order to verify the correct operation of at least a portion of said electromyograph.

15. The electromyograph of claim 14 wherein said elecW tromyograph is powered by direct current and wherein said 60 Hz reference signal generator comprises:

an antenna (200) adapted to pick up 60 Hz ambient electromagnetic radiation; and

frequency discriminating amplifier means (202-220) connected to said antenna for amplifying 60 Hz electromagnetic radiation by an amount greater than electromagnetic radiation at a frequency of other than 60 Hz, thereby generating said 60 Hz test signal.

16. The electromyograph of claim 1, further including calibration means for allowing the amplitude of said electromyograph signals to be accurately measured regardless of the gain of said electromyograph, said calibration means comprising:

a 60 Hz reference signal generator (200-220) producing a 60 Hz test signal having a known amplitude, said test signal being applied to an input of said amplifier means (204,206,140, 308); and

calculating means (530) receiving the digital data from said digital-to-analog converter means (520) for calculating the gain of each electromyograph channel from said amplifier means (204,206,140,308) to the output of said digital-to-analog converter means (520), thereby allowing said processing means (530,534) to multiply said digital data by a factor corresponding to said gain in order to accurately measure the amplitude of said electromyograph signals.

FIG. 1

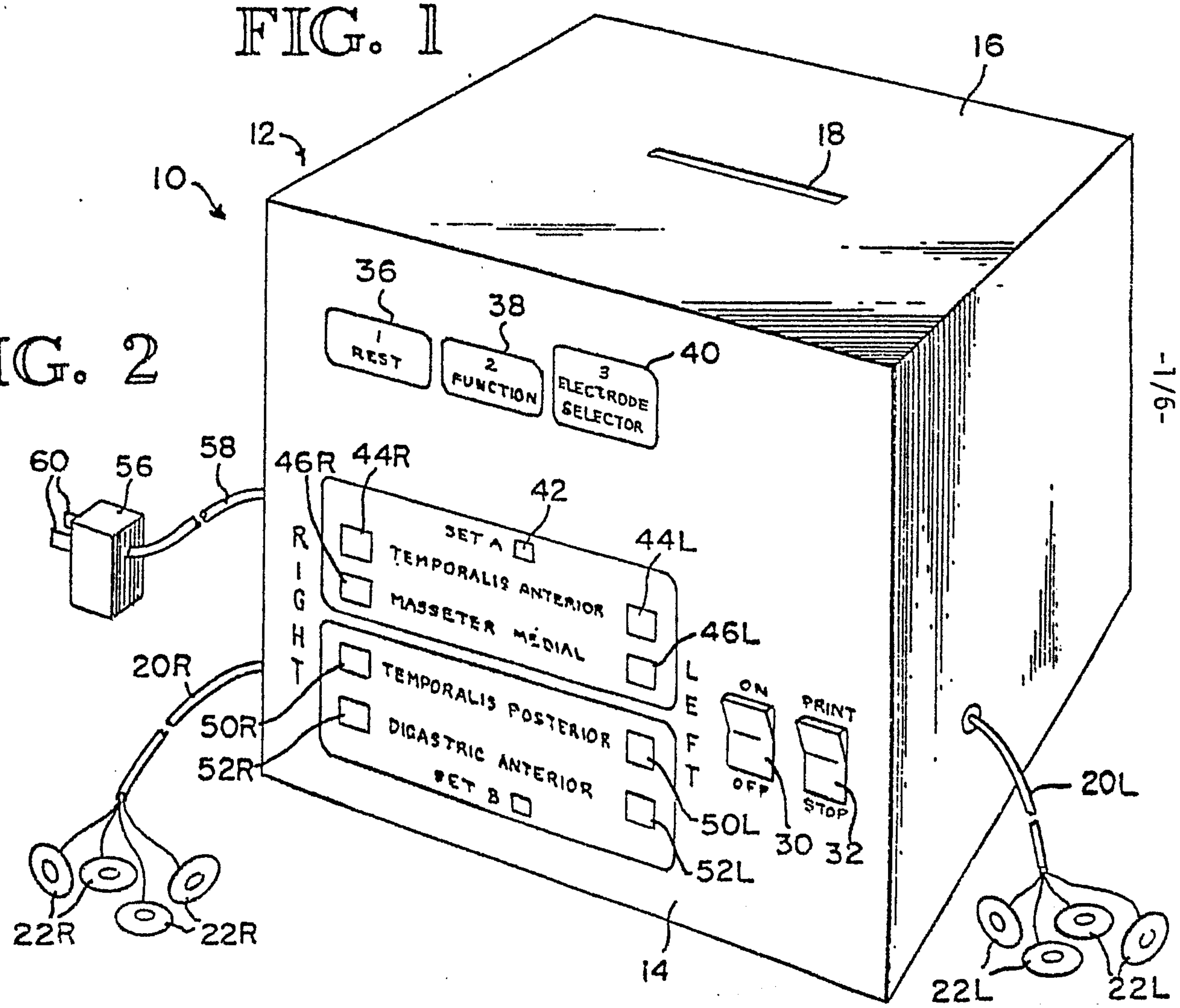


FIG. 2

ALL SYSTEMS OK — 70

NOISE TEST

3 5 3 3 — 72

PATIENTS NAME — 74

DATE __/__/__ — 76

EMG TEST 2 — 78

RELAX CLENCH RELAX — 80

| RIGHT | | LEFT | | |
|---|---|---|---|---|
| TA | MM | MM | TA | 82 |
| 1 | 2 | 1 | 2 | |
| 2 | 2 | 38 | 23 | |
| 3 | 6 | 223 | 32 | |
| 10 | 8 | 184 | 66 | |
| 14 | 11 | 213 | 161 | |
| 20 | 18 | 166 | 112 | |
| 5 | 3 | 147 | 48 | |
| 7 | 2 | 36 | 52 | |
| 9 | 2 | 70 | 95 | |
| 12 | 4 | 167 | 104 | 84 |
| 9 | 6 | 138 | 77 | 86 |

EMG TEST 1 — 90

MANDIBLE AT REST — 92

| RIGHT | | LEFT | | |
|---|---|---|---|---|
| TA | MM | MM | TA | 94 |
| 0 | 1 | 1 | 1 | |
| 3 | 25 | 25 | 6 | |
| 6 | 44 | 29 | 20 | |
| 5 | 47 | 78 | 27 | |
| 3 | 27 | 80 | 8 | |
| 1 | 3 | 13 | 4 | |
| 0 | 1 | 1 | 1 | |
| 0 | 1 | 0 | 1 | |
| 0 | 1 | 0 | 1 | 96 |

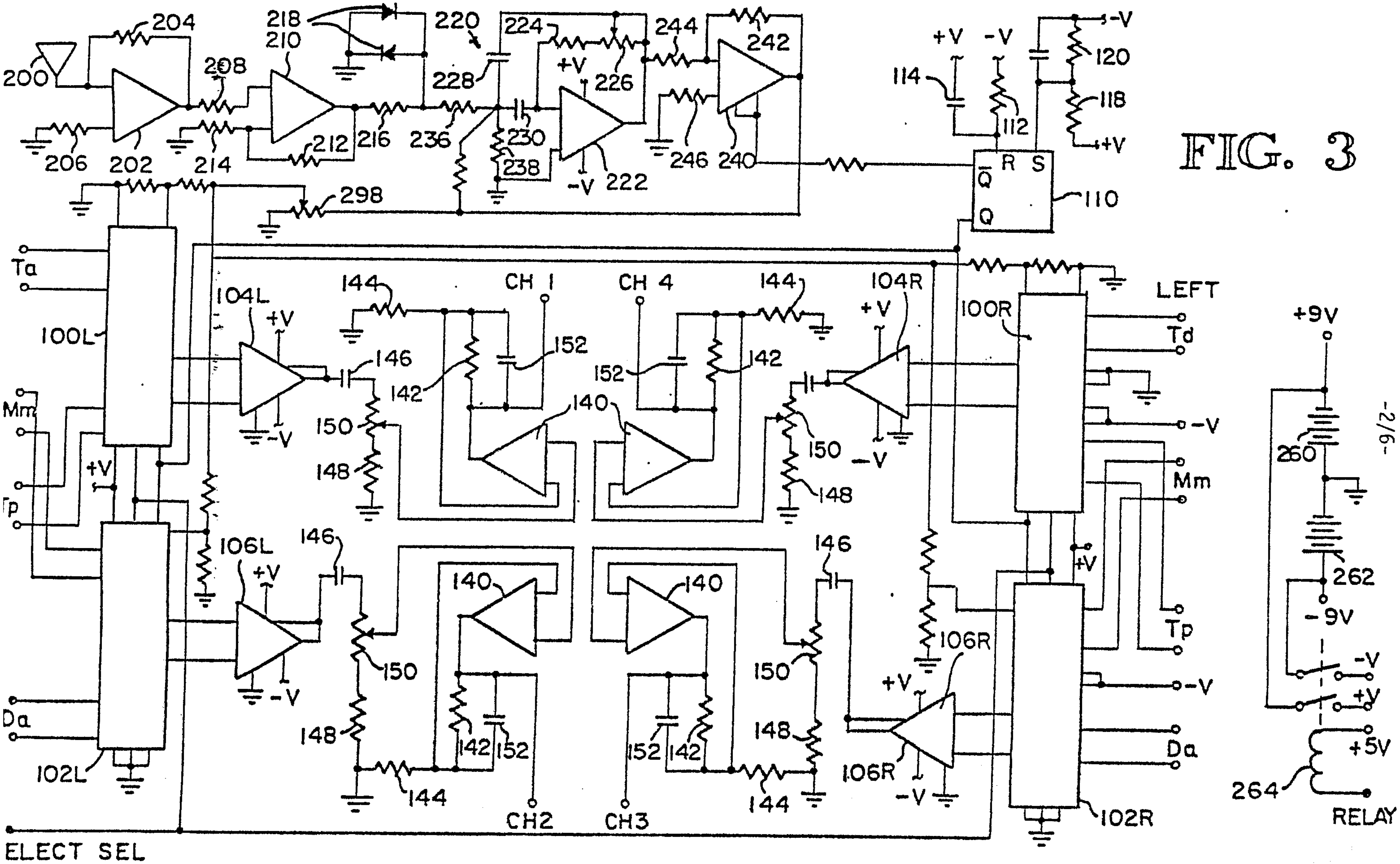

200
204
206
202
208
214
218
210
212
216
220
228
236
230
238
224
226
222
+V
-V
244
242
246
240
298
114
112
120
118
110
R
S
Q
100L
102L
104L
106L
104R
106R
100R
102R
140
142
144
146
148
150
152
CH 1
CH 2
CH 3
CH 4
Ta
Mm
Tp
Da
Td
LEFT
ELECT SEL
+9V
-9V
+5V
260
262
264
RELAY


FIG. 3

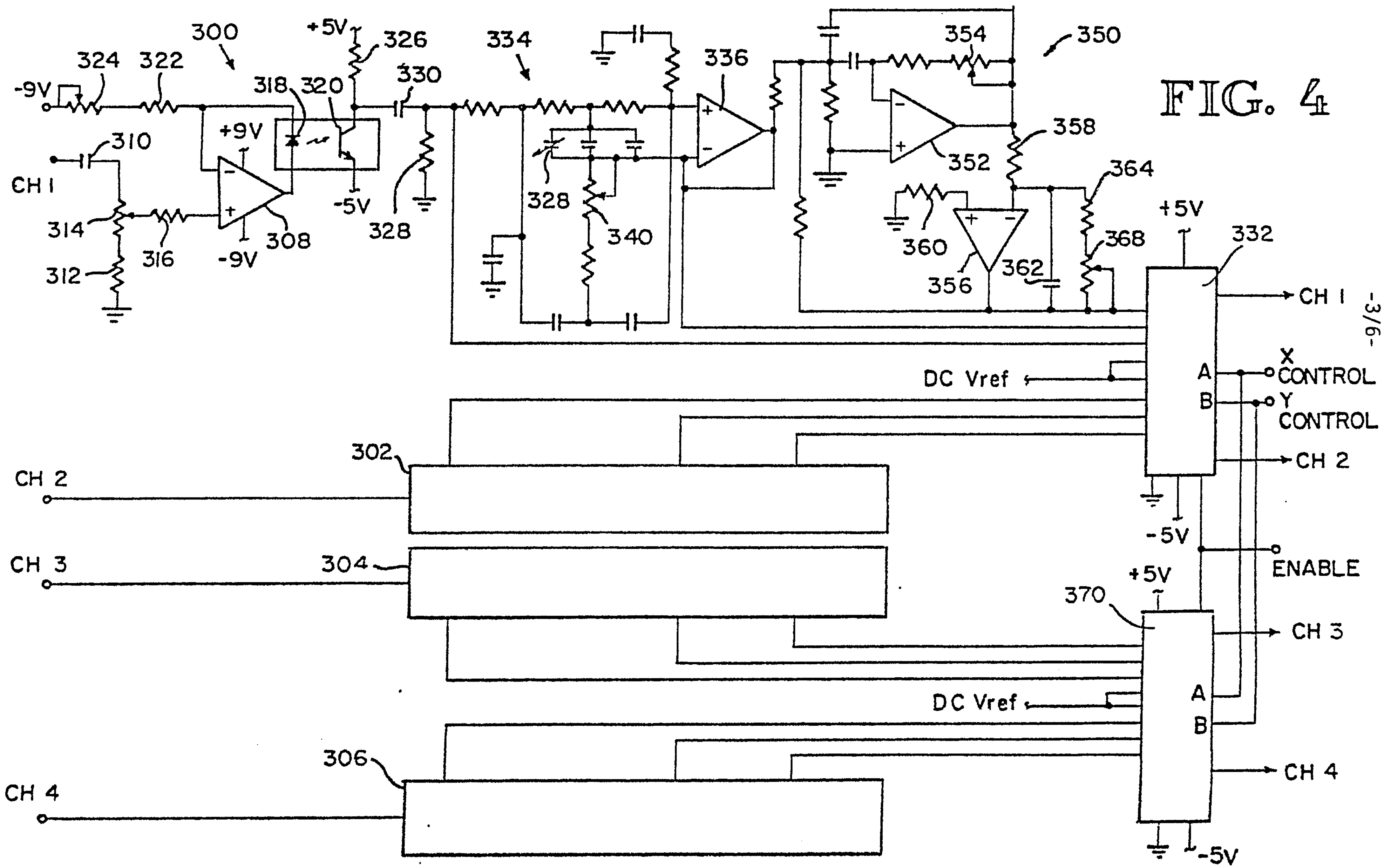
-9V
324
322
300
318
320
+5V
326
330
334
336
350
354
358
352
364
360
356
362
368
+9V
-9V
308
310
CH I
314
316
312
-5V
328
328
340
332
370
CH I
X CONTROL
Y CONTROL
A
B
DC Vref
CH 2
+5V
-5V
ENABLE
CH 3
CH 4
302
304
306
CH 2
CH 3
CH 4

FIG. 4

0108341

-4/6-

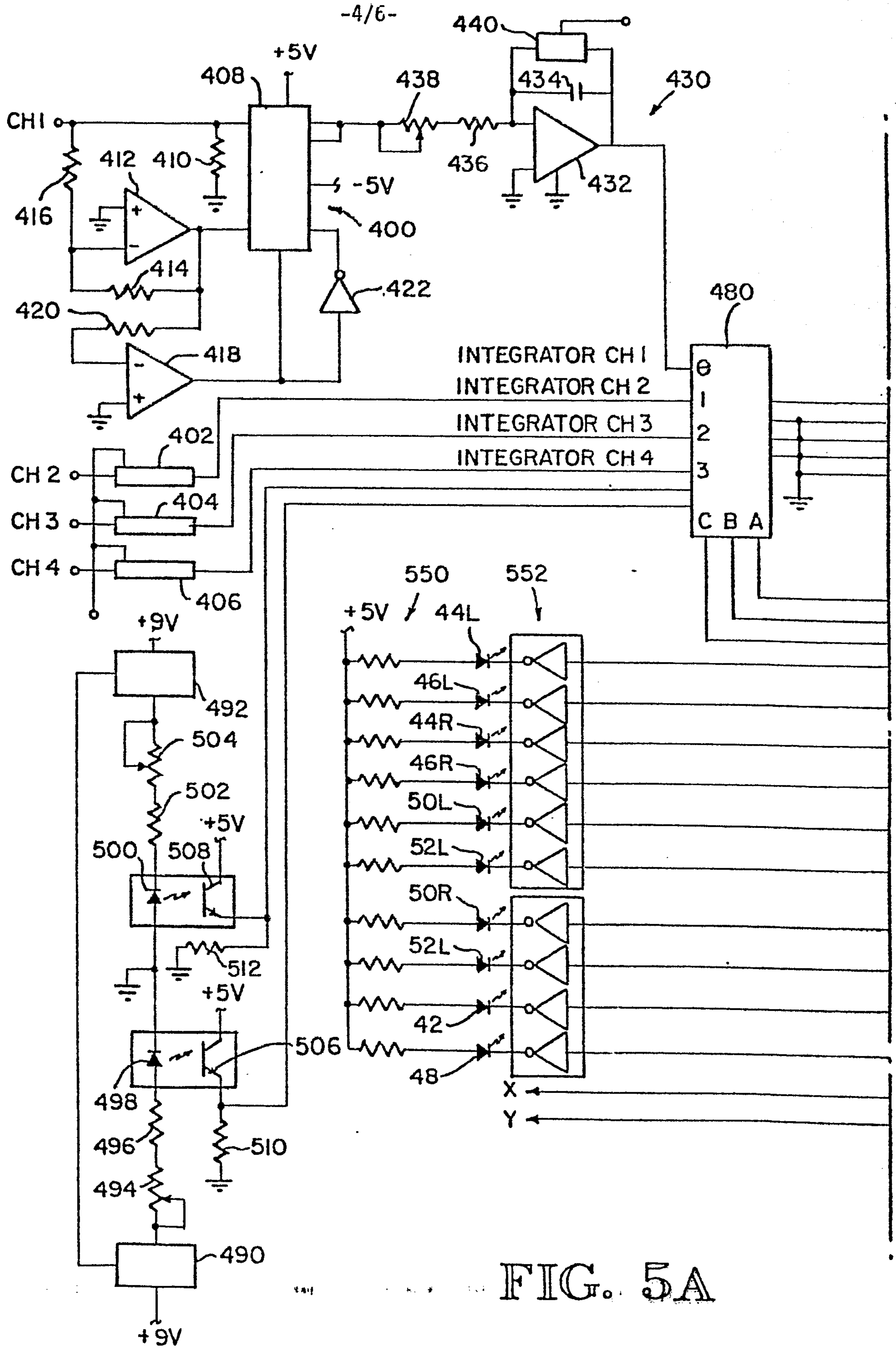


FIG. 5A

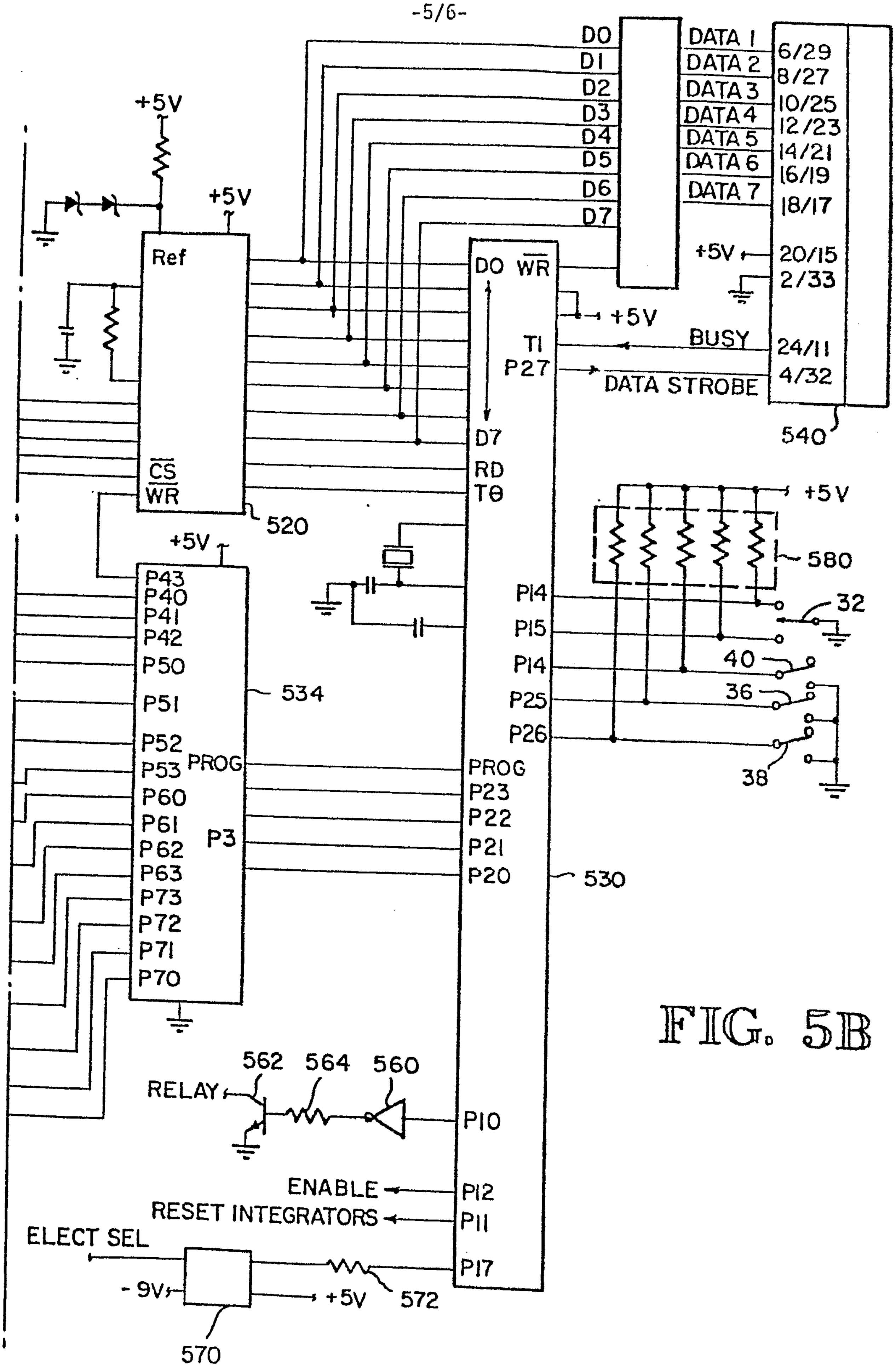

D0
D1
D2
D3
D4
D5
D6
D7
DATA 1
DATA 2
DATA 3
DATA 4
DATA 5
DATA 6
DATA 7
6/29
8/27
10/25
12/23
14/21
16/19
18/17
+5V
20/15
2/33
BUSY
24/11
DATA STROBE
4/32
540
+5V
Ref
+5V
CS
WR
520
D0 WR
T1
P27
D7
RD
T0
+5V
+5V
580
P14
P15
P14
P25
P26
32
40
36
38
+5V
P43
P40
P41
P42
P50
P51
534
P52
P53
PROG
P60
P61
P62
P3
P63
P73
P72
P71
P70
PROG
P23
P22
P21
P20
530
562
564
560
RELAY
P10
ENABLE
P12
RESET INTEGRATORS
P11
ELECT SEL
-9V
+5V
572
P17
570


FIG. 5B

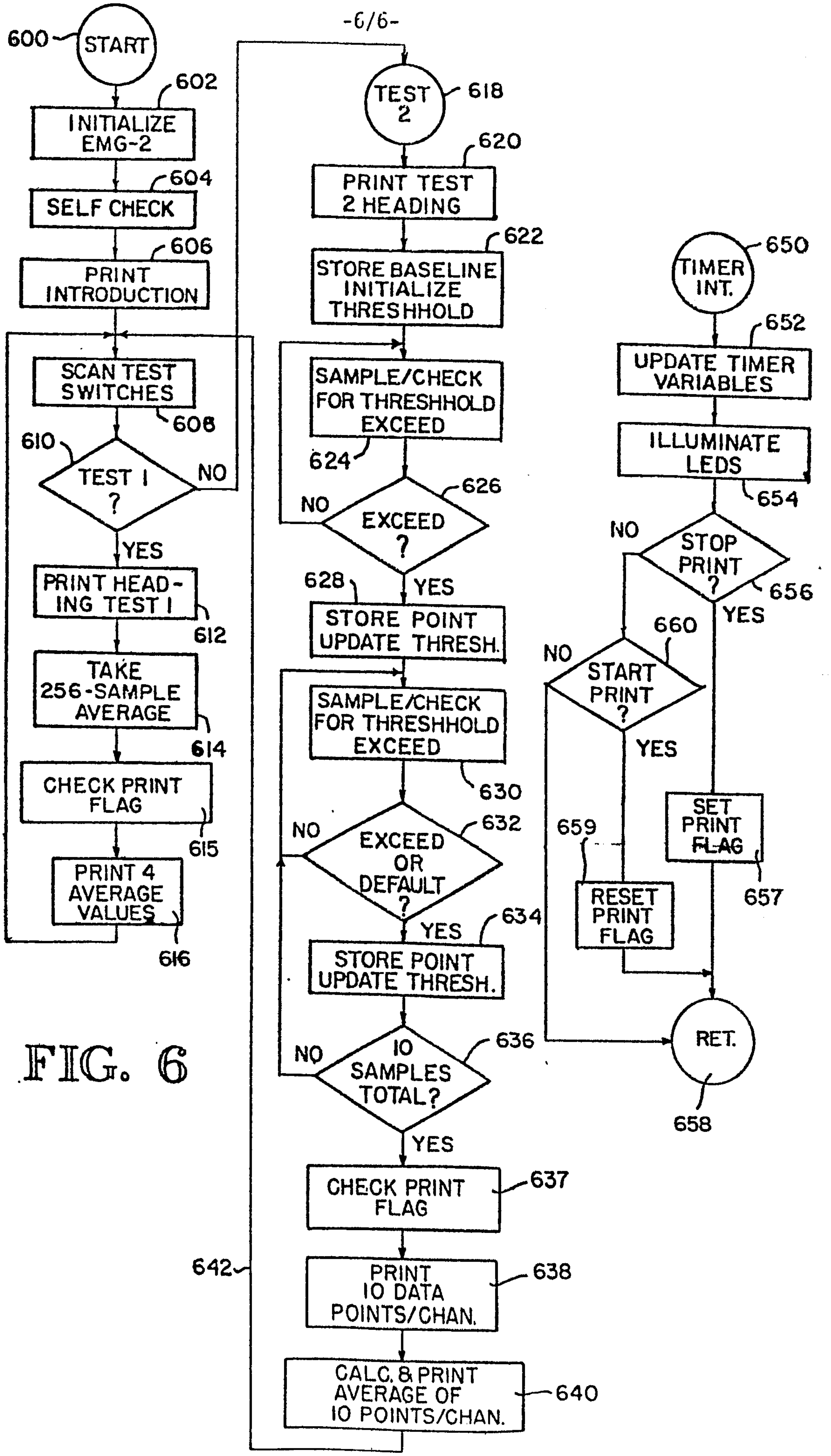
600
START
602
INITIALIZE EMG-2
604
SELF CHECK
606
PRINT INTRODUCTION
SCAN TEST SWITCHES
608
610
TEST I ?
NO
YES
PRINT HEAD-ING TEST I
612
TAKE 256-SAMPLE AVERAGE
614
CHECK PRINT FLAG
615
PRINT 4 AVERAGE VALUES
616
TEST 2
618
620
PRINT TEST 2 HEADING
622
STORE BASELINE INITIALIZE THRESHHOLD
SAMPLE/CHECK FOR THRESHHOLD EXCEED
624
626
NO
EXCEED ?
YES
628
STORE POINT UPDATE THRESH.
SAMPLE/CHECK FOR THRESHHOLD EXCEED
630
NO
632
EXCEED OR DEFAULT ?
YES
634
STORE POINT UPDATE THRESH.
NO
636
10 SAMPLES TOTAL?
YES
CHECK PRINT FLAG
637
642
PRINT 10 DATA POINTS/CHAN.
638
CALC. & PRINT AVERAGE OF 10 POINTS/CHAN.
640
TIMER INT.
650
652
UPDATE TIMER VARIABLES
ILLUMINATE LEDS
654
NO
STOP PRINT ?
656
YES
660
NO
START PRINT ?
YES
SET PRINT FLAG
657
659
RESET PRINT FLAG
RET.
658

FIG. 6